# EUROPÄISCHE PATENTSCHRIFT

(11) **EP 0 499 586 B1**
(45) Veröffentlichungstag und Bekanntmachung des Hinweises auf die Patenterteilung: **05.06.1996**
(21) Anmeldenummer: 92810085.8
(22) Anmeldetag: 06.02.1992
(51) Int. Cl.: A61K 31/445

(54) **Substituiertes Benzofuranylpiperidin als Nootropikum**
Substituted benzofuranyl-piperidine as nootropic
Benzofuranyl pipéridine substitué comme agent nootropique

(30) Priorität: 15.02.1991 CH 470/91
(43) Veröffentlichungstag der Anmeldung: 19.08.1992
(73) Patentinhaber: CIBA-GEIGY AG, CH-4002 Basel (CH)
(72) Erfinder: Mondadori, Cesare, Dr., CH-4147 Aesch (CH)

(56) Entgegenhaltungen:
- WO-A-89/03692
- DRUGS OF THE FUTURE, Band 10, Nr. 5, 1985; P.C. WALDMEIER et al., Seiten 371-373
- ACT. NERV. SUPER., Band 32, Nr. 3, 1990; K. NAHUNEK et al., Seiten 230-231
- JOURNAL OF NEURAL TRANSMISSIONS, [P-D Sect], Band 1, 1989; R. IHL et al., Seiten 82-83
- DRUG NEWS PERSPECT, Band 3, Nr. 10, Dezember 1990; E. KYBURZ, Seiten 592-599
- PSYCHOPHARMACOLOGY, Band 91, Nr. 4, 1987; P.N. TARIOT et al., Seiten 489-495

## Beschreibung

Die Erfindung betrifft die Verwendung von 4-(7-Brom-5-methoxy-benzofuran-2-yl)piperidin der Formel I 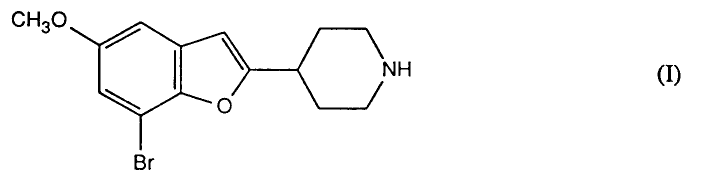
oder eines seiner pharmazeutisch verwendbaren Salze zur Herstellung eines Nootropikums, und für die Behandlung von mit degenerativen Nervenerkrankungen einhergehenden Degeneration von Nervenzellen.

Pharmazeutisch verwendbare Salze von 4-(7-Brom-5-methoxy-benzofuran-2-yl)piperidin sind insbesondere seine pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten aliphatischen oder aromatischen Sulfonsäuren oder N-substituiexen Sulfaminsäuren, z.B. Methansulfonate, Benzolsulfonate, p-Toluolsulfonate oder N-Cyclohexylsulfaminate (Cyclamate).

4-(7-Brom-5-methoxy-benzofuran-2-yl)piperidin und seine pharmazeutisch verwendbaren Salze sind bekannt. Sie hemmen an der Ratte nach subkutaner Verabreichung im Dosisbereich von etwa 10 bis etwa 50 mg/kg die Aufnahme von Noradrenalin ins Herz und die Monoaminooxydase und wurden dementsprechend als antidepressive Arzneimittelwirkstoffe zur Behandlung von Gemütsdepressionen vorgeschlagen.

Der Erfindung liegt die überraschende Feststellung zu Grunde, das 4-(7-Brom-5-methoxy-benzofuran-2-yl)piperidin und seine pharmazeutisch verwendbaren Salze zusätzlich ausgeprägte nootrope Wirkungen und eine ausgeprägte verlangsamende Wirkung auf die Degeneration von Nervenzellen, wie sie z.B. bei der Alzheimer'schen und der Parkinson'schen Krankheit auftreten, aufweisen. So bewirken sie an der Maus einen sicheren Schutz vor der amnesiogenen Wirkung eines cerebralen Elektroschocks und eine deutlich Verbesserung der Gedächtnisleistung. So zeigte sich nach peroraler Verabfolgung einer Dosis von etwa 0,1 bis etwa 3 mg/kg p.o. eine gegenüber Vehikel allein hochsignikikante Verlängerung der Verweildauer im hellen Testkäfigteil (Vehikel: 10,4 s; 3,0 mg/kg Wirkstoff in Form des Hydrochlorides: 38,8 ± 7,9 sec; p < 0,0002).

Zudem erleichtem sie im One-way Active Avoidance-Test, der an 27 Monate alten Ratten durchgeführt wurde, nach peroraler Verabfolgung einer Dosis von 0,3 und 3,0 mg/kg das Erlernen der Vemeidungsaufgabe, d.h. verringerten die Anzahl der für die Erreichung von 5 aufeinanderfolgenden aktiven Vermeidungreaktionen erforderlichen Lernversuche gegenüber Vehikel in signifikanter Weise (Vehikel: 21,7 ± 4,5; 3,0 mg/kg Wirkstoff in Form des Hydrochlorides: 10,2 ± 1,8; p < 0,05).

Auf Grund dieser Eigenschaften eignen sich 4-(7-Brom-5-methoxy-benzofuran-2-yl)piperidin und seine pharmazeutisch verwendbaren Salze vorzüglich zur Verlangsamung der mit degenerativen Nervenerkrankungen, wie der Alzheimer'schen und der Parkinson'schen Krankheit, einhergehenden Degeneration von Nervenzellen sowie zur Behandlung von auf Nootropikabehandlung ansprechenden Erkrankungen, beispielsweise von cerebralen Leistungsinsuffizienzen verschiedenaxigster Genese und Äthiologie, insbesondere von Gedächtnisstörungen, wie seniler Demenz, Multiinfarkt-Demenz oder Demenz vom Alzheimer-Typ, ferner von Hirnschlag sowie von Folgeerscheinungen von Hirntraumata und Apoplexien.

Die erfindungsgemäss vorgeschlagenen nootropen Arzneimittelwirkstoffe können enteral oder parenteral, insbesondere oral oder intravenös, verabreicht werden. Die empfohlene Tagesdosis beträgt beispielsweise etwa 0,6 bis etwa 18 mg/kg bzw. etwa 40 bis etwa 1250 mg/70 kg, vorzugsweise etwa 2 bis 8 mg/kg bzw. etwa 150 bis 600 mg/70 kg, beispielsweise etwa 2,5 bis etwa 6 mg/kg bzw. etwa 175 bis 420 mg/70 kg, die in erforderlichenfalls in 2 bis 4 Einzeldosis von beispielsweise etwa 0,3 bis etwa 6 mg/kg bzw. etwa 20 bis etwa 400 mg/70 kg, |vorzugsweise etwa 2 bis 4 mg/kg bzw. etwa 150 bis 300 mg/70 kg, beispielsweise etwa 2,5 bis etwa 3,2 mg/kg bzw. etwa 175 bis 225 mg/70 kg aufgeteilt werden kann.

Bei den erfindungsgemäss bereitgestellten nootropen pharmazeutischen Präparaten handelt es sich vorzugsweise um solche in einer Dosiseinheitsform zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist.

Die erfindungsgemäss bereitgestellten nootropen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaftresistenten Überzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemische oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetisch Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Äthyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die nachfolgenden Beispiele dienen zur Illustration der Erfindung; Temperaturen sind in Celsiusgraden, Drucke in mbar angegeben.

### Pharmakologisches Beispiel 1: Schutz vor der amnesiogen Wirkung eines cerebralen Elektroschocks an der Maus

Es ist klinische Erfahrungstatsache, dass cerebrale Elektroschockbehandlung zu retrograder Amnesie führt; das Erinnerungsvermögen an Ereignisse unmittelbar vor dem Elektroschock wird gestört. Weder der biochemische Mechanismus noch die Art des durch die Behandlung beeinflussten psychologischen Prozesse ist bekannt. Es besteht jedoch kaum Zweifel daran, dass der Elektroschock den Erinnerungsprozess irgendwie beeinflusst. Die Zeitabhängigkeit der retrograden Amnesie (je weiter die Informationsaufnahme zurückliegt, desto geringer ist der Effekt) spricht für eine Einwirkung auf einen zeitlich begrenzten Prozess, der wahrscheinlich am ehesten die der Fixierung der Gedächtnisspur oder Verfestigung des Geächtnisinhaltes betrifft. Es ist deshalb anzunehmen, dass Arzneimittel mit die Fixierung der Gedächtnisspur erleichternder Wirkung die durch den Elektroschock bewirkte Amnesie günstig beeinflussen, beispielsweise die Zeitspanne während der die Fixierung der Gedächtnisspur gestört wird, verkürzen und so das Ausmass der Amnesie vermindern. Im Tierexperiment kann eine durch Elektroschock induzierte Amnesie festgestellt werden, wenn der cerebrale Elektroschock innerhalb weniger Sekunden nach dem Lernvorgang appliziert wird. Dieser besteht im vorliegenden Fall in einer passiven Vermeidungsaufgabe.

### Versuchanordnung:

Ein grösserer Käfigteil (35 x 20 x 10 cm) ist mit einem kleineren Käfigteil (10 x 10 x 10 cm) durch eine Schiebetür verbunden. Der kleinere Käfigteil wird von oben mit einer 100 Watt starken Glühlampe beleuchtet, der grössere Käfigteil ist dunkel. Der Boden beider Käfigteile besteht aus einem elektrisch leitenden Gitter.

### Methodik:

Die Versuchstiere werden einzeln in den hellen Käfigteil gesetzt. Da Mäuse eine natürliche Vorliebe für dunkle Umgebung besitzen, begeben sich die meisten Versuchstiere spontan in den dunklen Käfigteil. Sobald sich alle Versuchstiere in diesem befinden, wird die Schiebetür geschlossen und ein Fußschock (1 mA, 5 sec) verabfolgt.

Die Versuchtiere werden sofort danach entnommen und nach 24 Stunden erneut in den hellen Käfigteil gesetzt. Die Zeitspanne, die die Versuchstiere zögern, bis sie sich in den dunklen Käfigteil begeben, wird gemessen. Im allgemeinen verbleiben die meisten Versuchstiere die gesamte Beobachtungsdauer (150 sec) im hellen Käfigteil, was bedeutet, das praktisch alle Versuchstiere die Lernaufgabe bewältigt haben.

Wird unmittelbar nach der Fusschockphase des Lernprozesses ein cerebraler Elektroschock (14 mA, 0,2 s, 150 Hz) verabfolgt, wird das Erinnerungsvermögen an den Fußschock gestört. 4-(7-Brom-5-methoxy-benzofuran-2-yl)piperidin in Form des Hydrochlorides hat in diesem Modell nach peroraler Verabreichung 60 Minuten vor dem cerebralen Elektroschock eine ausgeprägte antiamnesiogene Schutzwirkung

### Messergebnisse:

| Testsubstanz | Verweildauer im hellen Käfigteil | Signifikanz |
|---|---|---|
| 0.3 mg/kg p.o. Wirkstoff | 25,9 s | p<0,01 |
| 3.0 mg/kg p.o. Wirkstoff | 38,9 s | p<0,0002 |
| 30.0 mg/kg p.o. Wirkstoff | 14,5 s | n.s. |
| Vehikel allein | 10,4 s | |

### Schlussfolgerung:

Der Wirkstoff ist wirksam bei 0,3, 1,0 und 3.0 mg/kg p.o. Die stärkste Wirkung ist bei 3,0 mg/kg p.o. zu beobachten; die durchschnittliche Verweildauer in hellen Käfigteil beträgt dann 38,8 ± 7,9 sec (p < 0,0002). Bei 30,0 mg /kg p.o. wird keine signifikante Wirkung mehr festgestellt. Gegenüber der Verweildauer nach Vehikel allein (10,4 s) entspricht dies einer statistisch hochsignifikanten Verlängerung um den Faktor 3,7.

### Pharmakologisches Beispiel 2: Wirkung bei chronischer Behandlung auf die Lernfähigkeit alter Ratten

Eine gewisse Abnahme höherer geistiger Funktionen, feststellbar vor allem bei der Speicherung und Verwertung von Informationen, ist bei Mensch und Tier Merkmal und Folge des natürlichen Alterungsprozesses. Am Tier kann eine Abnahme der Fähigkeit festgestellt werden, Informationen zu sammeln und zu verwerten. Alte Versuchstiere sind dementsprechend brauchbare Modellobjekte für die Untersuchung altersbedingter kognitiver Fehlfunktionen und insbesondere von Arzneimittelwirkstoffen auf altersbedingte Gedächtnisfehlleistungen.

### Versuchsanordnung:

Die Versuchanordnung besteht aus 2 gleichen durch eine Tür (12 x 16 cm) miteinander verbundenen und mit elektrisierbaren Bodengittern versehenen Käfigabteilen A und B (je 20 x 20 x 30 cm). Der Lernversuch besteht darin, dass das Versuchstier in Käfigteil A gesetzt und nach 10 Sekunden einem elektrischen Fußschock unterworfen wird. Das Versuchstier kann diesen vermeiden, indem es sich in den Käfigteil B begibt. Das Lerntraining in dieser aktiven Einwegvermeidungsaufgabe wird solange fortgesetzt, bis das Versuchstier 5-mal nacheinander den Elektroschock auf diese Weise zu vermeiden in der Lage ist.

### Methode:

Alte Ratten (Alter bei Versuchsbeginn: 27 Monate) werden täglich peroral mit 0,3, 1,0, 3,0 und 30 mg/kg der Testsubstanz behandelt. Einer Kontrollgruppe wird das Vehikel verabreicht. 60 Minuten nach Behandlung werden die Versuchtiere dem obigen Lerntraining unterworfen. Ein weiterer Lernversuch erfolgt 4 Stunden später.

### Messergebnisse:

| Testsubstanz | Anzahl der Lernversuche | Signifikanz |
|---|---|---|
| 0,3 mg /kg p.o. Wirkstoff | 15,6 ± 2,6 | p<0,05 |
| 3.0 mg/kg p.o. Wirkstoff | 10,2 ± 1,8 | p<0.02 |
| Vehikel allein | 21,7 ± 4,5 | |

### Schlussfolgerung:

Eine Dosis von 0,3 und 3,0 mg/kg p.o. des Wirkstoffes verringert die für das Erlernen der aktiven Vermeidungsaufgabe nötigen Lernversuche in statistisch signifikanter Weise auf 47 % des Kontrollwertes.

### Formulierungsbeispiel 1:

Tabletten, enthaltend je 200 mg 4-(7-Brom-5-methoxy-benzofuran-2-yl)piperidin oder ein Salz, z.B. das Hydrochlorid, davon, können wie folgt hergestellt werden:

### Zusammensetzung (10000 Tabletten)

| | |
|---|---|
| Wirkstoff | 2000,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Äthanol | q.s. |

Der Wirkstoff wird mit der Lactose und 292 g Kartoffelstärke vermischt, die Mischung mit einer äthanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 295,0 mg Gewicht und 50,0 mg Wirkstoffgehalt, die gewünschtenfalls mit Teilkerben zur feineren Anpassung der Dosierung versehen sein können.

### Formulierungsbeispiel 2:

Lacktabletten, enthaltend je 400 mg 4-(7-Brom-5-methoxy-benzofuran-2-yl)piperidin oder ein Salz, z.B. das Hydrochlorid, davon, können wie folgt hergestellt werden:

### Zusammensetzung (für 1000 Lacktabletten)

| | |
|---|---|
| Wirkstoff | 400,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 580 mg) verpresst und diese mit einer Lösung der Hydroxypropylmethylcellulose und des Schellacks in Methylenchlorid lackiert; Endgewicht der Lacktablette: 583 mg.

### Formulierungsbeispiel 3:

Gelatinesteckkapseln, enthaltend 500 mg 4-(7-Brom-5-methoxy-benzofuran-2-yl)piperidin oder ein Salz, z.B. das Hydrochlorid, davon, können z.B. folgendermassen hergestellt werden:

### Zusammensetzung (für 1000 Kapseln)

| | |
|---|---|
| Wirkstoff | 500,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 790 mg der erhaltenen Formulierung in Gelatinesteckkapseln passender Grösse abgefüllt.

### Formulierungsbeispiel 4:

Eine 5%-ige Injektions- oder Infusionslösung von 4-(7-Brom-5-methoxy-benzofuran-2-yl)piperidin oder eines Salzes, z.B. des Hydrochlorides, davon, kann beispielsweise folgendermassen hergestellt werden:

### Zusammensetzung (für 1000 bzw. 400 Ampullen)

| | |
|---|---|
| Wirkstoff | 125,0 g |
| Natriumchlorid | 22,5 g |
| Phosphatpuffer pH= 7.4 | 300,0 g |
| entmineralisiertes Wasser | ad 2500,0 ml |

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 50 bzw. 125 mg Wirkstoff enthalten.

## Patentansprüche (Patentansprüche für folgende(n) Vertragsstaat(en): AT, BE, CH, DE, DK, FR, GB, IT, LI, LU, NL, PT, SE)

1. Verwendung von 4-(7-Brom-5-methoxy-benzofuran-2-yl)piperidin der Formel I 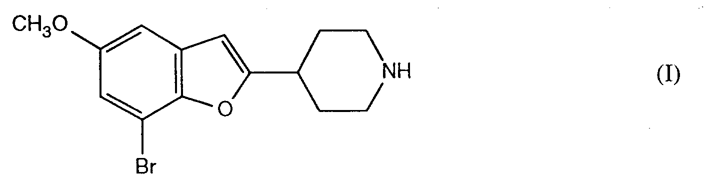 oder eines seiner pharmazeutisch verwendbaren Salze für die Herstellung eines Arzneimittels für die Behandlung von mit degenerativen Nervenerkrankungen einhergehenden Degeneration von Nervenzellen sowie als Nootropikum.

## Patentansprüche (Patentansprüche für folgende(n) Vertragsstaat(en): ES, GR)

1. Verfahren zur Herstellung eines für die Verlangsamung der mit degenerativen Nervenerkrankungen einhergehenden Degeneration von Nervenzellen sowie als Nootropikum bestimmten pharmazeutischen Präparates, dadurch gekennzeichnet, dass man 4-(7-Brom-5-methoxy-benzofuran-2-yl)piperidin der Formel I 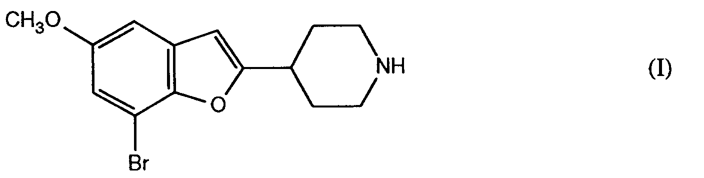 oder eines seiner pharmazeutisch verwendbaren Salzemit üblichen pharmazeutischen Hilfsstoffen vermischt.

2. Verfahren gemäss Anspruch 1, dadurch gekennzeichnet, dass man ein pharmazeutisches Präparat in einer Dosiseinheitsform herstellt.

3. Verfahren gemäss einem der Ansprüche 1 und 2, dadurch gekennzeichnet, dass man ein pharmazeutisches Präparat in einer enteralen, wie oralen, Dosiseinheitsform herstellt.

4. Verfahren gemäss einem der Ansprüche 1 bis 3, dadurch gekennzeichnet, dass man Tabletten, Lacktabletten, Gelatinekapseln, Steckkapseln oder Dragées herstellt.

5. Verfahren gemäss einem der Ansprüche 1 und 2, dadurch gekennzeichnet, dass man ein pharmazeutisches Präparat in einer für die parenterale, wie intravenöse oder intraperitoneale, Verabreichung geeigneten Darreichungsform herstellt.

6. Verfahren gemäss Anspruch 5, dadurch gekennzeichnet, dass man Injektions- oder Infusionslösungen herstellt.

7. Verfahren gemäss einem der Ansprüche 1 bis 6, dadurch gekennzeichnet, dass man ein pharmazeutisches Präparat herstellt, welches pro Dosiseinheit enthaltend etwa 20 bis etwa 400 mg Wirkstoff enthält.

8. Verfahren gemäss einem der Ansprüche 1 bis 6, dadurch gekennzeichnet, dass man ein pharmazeutisches Präparat herstellt, welches pro Dosiseinheit enthaltend etwa 150 bis etwa 300 mg Wirkstoff enthält.

9. Verfahren gemäss einem der Ansprüche 1 bis 6, dadurch gekennzeichnet, dass man ein pharmazeutisches Präparat herstellt, welches pro Dosiseinheit enthaltend etwa 175 bis etwa 225 mg Wirkstoff enthält.

10. Verfahren gemäss einem der Ansprüche 1, 2 und 6 bis 9, dadurch gekennzeichnet, dass man eine Injektions- oder Infusionslösungen mit einem Wirkstoffgehalt von etwa 1 bis etwa 10, insbesondere 2,5 bis 10 Gew.-%, herstellt.

## Claims (Claims for the following Contracting State(s): AT, BE, CH, DE, DK, FR, GB, IT, LI, LU, NL, PT, SE)

1. The use of 4-(7-bromo-5-methoxybenzofuran-2-yl)-piperidine of formula I 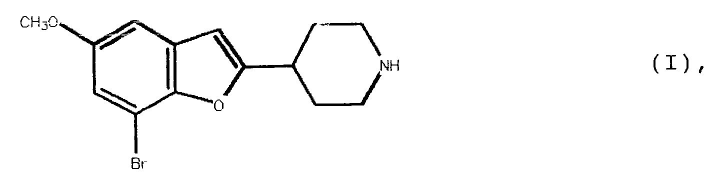 or of a pharmaceutically acceptable salt thereof, in the preparation of a medicament for treating the degeneration of nerve cells that accompanies degenerative nerve disorders, and as a nootropic agent.

## Claims (Claims for the following Contracting State(s): ES, GR)

1. A process for the preparation of a pharmaceutical composition for retarding the degeneration of nerve cells that accompanies degenerative nerve disorders, and for use as a nootropic agent, which comprises mixing 4-(7-bromo-5-methoxybenzofuran-2-yl)piperidine of formula I 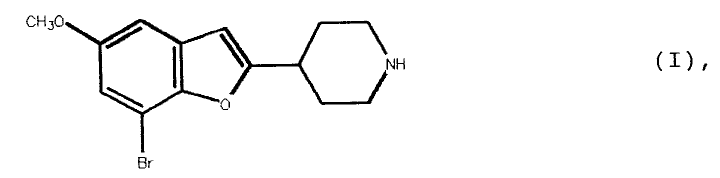 or a pharmaceutically acceptable salt thereof, with customary pharmaceutical excipients.

2. A process according to claim 1, wherein there is prepared a pharmaceutical composition in unit dose form.

3. A process according to either claim 1 or claim 2, wherein there is prepared a pharmaceutical composition in an enteral, such as oral, unit dose form.

4. A process according to any one of claims 1 to 3, wherein there is prepared a tablet, film-coated tablet, gelatin capsule, hard gelatin capsule or dragée.

5. A process according to either claim 1 or claim 2, wherein there is prepared a pharmaceutical composition in a dosage form suitable for parenteral, such as intravenous or intraperitoneal, administration.

6. A process according to claim 5, wherein there is prepared an injection or infusion solution.

7. A process according to any one of claims 1 to 6, wherein there is prepared a pharmaceutical composition comprising from approximately 20 to approximately 400 mg of active ingredient per unit dose.

8. A process according to any one of claims 1 to 6, wherein there is prepared a pharmaceutical composition comprising from approximately 150 to approximately 300 mg of active ingredient per unit dose.

9. A process according to any one of claims 1 to 6, wherein there is prepared a pharmaceutical composition comprising from approximately 175 to approximately 225 mg of active ingredient per unit dose.

10. A process according to any one of claims 1, 2 and 6 to 9, wherein there is prepared an injection or infusion solution comprising from approximately 1 to approximately 10 % by weight, especially from 2.5 to 10 % by weight, active ingredient.

## Revendications (Revendications pour l'(les) Etat(s) contractant(s) suivant(s): AT, BE, CH, DE, DK, FR, GB, IT, LI, LU, NL, PT, SE)

1. Utilisation de la 4-(7-bromo-5-méthoxybenzofuranne-2-yl)-pipéridine de formule I 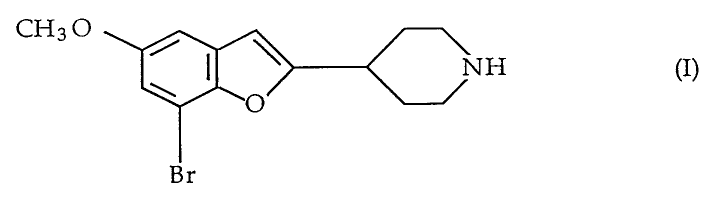 ou de l'un de ses sels acceptables pour l'usage pharmaceutique pour la préparation d'un médicament prévu pour le traitement de la dégénération des cellules nerveuses accompagnant les maladies nerveuses dégénératives et en tant que médicament nootropique.

## Revendications (Revendications pour l'(les) Etat(s) contractant(s) suivant(s): ES, GR)

1. Procédé de préparation d'une composition pharmaceutique servant à ralentir la dégénération des cellules nerveuses accompagnant les maladies nerveuses dégénératives ou utilisable en tant qu'agent nootropique, caractérisé en ce que l'on mélange la 4-(7-bromo-5-méthoxybenzofuranne-2-yl)-pipéridine de formule I 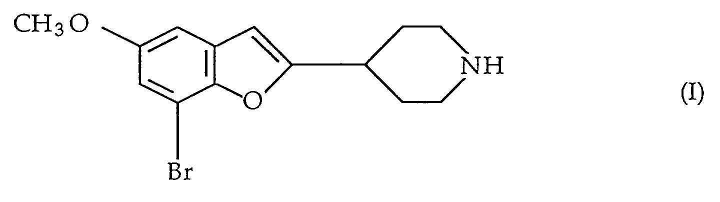 ou l'un de ses sels acceptables pour l'usage pharmaceutique, avec des produits auxiliaires pharmaceutiques usuels.

2. Procédé selon la revendication 1, caractérisé en ce que l'on prépare une composition pharmaceutique à l'état d'unités de dosage.

3. Procédé selon l'une des revendications 1 et 2, caractérisé en ce que l'on prépare une composition pharmaceutique à l'état d'unités de dosage entérales, par exemple orales.

4. Procédé selon l'une des revendications 1 à 3, caractérisé en ce que l'on prépare des comprimés, des comprimés revêtus, des capsules de gélatine, des gélules ou des dragées.

5. Procédé selon l'une des revendications 1 et 2, caractérisé en ce que l'on prépare une composition pharmaceutique sous une forme d'administration appropriée à l'administration parentérale, par exemple intraveineuse ou intrapéritonéale.

6. Procédé selon la revendication 5, caractérisé ce que l'on prépare des solutions pour injections ou perfusions.

7. Procédé selon l'une des revendications 1 à 6, caractérisé en ce que l'on prépare une composition pharmaceutique contenant d'environ 20 à 400 mg de substance active par unité de dosage.

8. Procédé selon l'une des revendications 1 à 6, caractérisé en ce que l'on prépare une composition pharmaceutique contenant d'environ 150 à 300 mg de substance active par unité de dosage.

9. Procédé selon l'une des revendications 1 à 6, caractérisé en ce que l'on prépare une composition pharmaceutique contenant d'environ 175 à 225 mg de substance active par unité de dosage.

10. Procédé selon l'une des revendications 1, 2 et 6 à 9 caractérisé en ce que l'on prépare des solutions pour injections ou perfusions à une teneur en substance active d'environ 1 à 10 et plus spécialement de 2,5 à 10 % en poids.
